(19) Europäisches Patentamt
European Patent Office
Office européen des brevets

(11) Veröffentlichungsnummer: **0 052 742**
**A1**

(12) # EUROPÄISCHE PATENTANMELDUNG

(21) Anmeldenummer: **81108209.8**

(22) Anmeldetag: **12.10.81**

(51) Int. Cl.³: **C 07 C 131/00**

(30) Priorität: **26.11.80 DE 3044517**

(43) Veröffentlichungstag der Anmeldung:
**02.06.82 Patentblatt 82/22**

(84) Benannte Vertragsstaaten:
**BE DE FR GB IT**

(71) Anmelder: **BASF Aktiengesellschaft**
**Carl-Bosch-Strasse 38**
**D-6700 Ludwigshafen(DE)**

(72) Erfinder: **Schaefer, Peter, Dr.**
**Im Buegen 16**
**D-6719 Kirchheim(DE)**

(72) Erfinder: **Mangold, Dietrich, Dr.**
**Hermann-Walker-Strasse 49**
**D-6903 Neckargemuend(DE)**

(54) **Verfahren zur Herstellung von alpha-Chlorketoximethern.**

(57) Herstellung von α-Chlorketoximethern durch Umsetzung von α-Chlorketonen mit Oximethern in Gegenwart von Säure.

Die nach dem Verfahren der Erfindung herstellbaren α-Chlorketoximethern sind wertvolle Ausgangsstoffe für die Herstellung von Farbstoffen, Pharmazeutika und Schädlingsbekämpfungsmitteln.

EP 0 052 742 A1

# Verfahren zur Herstellung von α-Chlorketoximethern

Die vorliegende Erfindung betrifft ein neues Verfahren zur Herstellung von α-Chlorketoximethern durch Umsetzung von α-Chlorketonen mit Oximethern in Gegenwart von Säure.

α-Chloracetophenonoximether sind in DE-OS 29 07 972 beschrieben. Sie werden durch Kondensation von α-Chloracetophenonen mit O-substituierten, N-unsubstituierten Hydroxylaminen in Gegenwart eines Verdünnungsmittels hergestellt. Nachteil dieses Verfahrens ist die aufwendige Herstellung von O-substituierten Hydroxylaminen. So wird in einer ersten Reaktion das Stickstoffatom des Hydroxylamins durch Einführung einer Schutzgruppe blockiert, in einer zweiten Reaktion erfolgt die Alkylierung am Sauerstoffatom und in einer dritten Reaktion wird die Schutzgruppe am Stickstoffatom wieder abgespalten (Houben-Weyl, Methoden der Organischen Chemie, Band 10/1, Seite 1 181). Die Zwischenprodukte müssen jeweils isoliert und gereinigt werden, da die folgende Umsetzung nur in einem anderen Lösungsmittel optimal verläuft, wodurch ein mehrstufiges Verfahren resultiert. Hinzu kommen die oft nicht befriedigenden Ausbeuten. Das Verfahren ist in seiner Gesamtheit unwirtschaftlich.

Es wurde nun gefunden, daß man α-Chlorketoximether der Formel

$$R^1 - \overset{NOR^3}{\overset{\|}{C}} - \underset{Cl}{\overset{R^2}{C}} - R^2 \qquad I,$$

worin $R^1$, $R^2$ und $R^3$ gleich oder verschieden sein können und jeweils einen aliphatischen oder cycloaliphatischen Rest bedeuten, $R^1$ und $R^2$ auch jeweils einen gegebenenfalls durch Halogenatome substituierten Arylrest bezeichnen, oder $R^1$

WB/BL

und ein Rest $R^2$ zusammen mit den beiden benachbarten Kohlenstoffatomen für Glieder eines alicyclischen Ringes stehen können, $R^3$ auch einen araliphatischen Rest bedeutet, $R^2$ auch für ein Wasserstoffatom steht, vorteilhaft erhält, wenn man α-Chlorketone der Formel

$$R^1 - \overset{O}{\overset{\|}{C}} - \underset{Cl}{\overset{R^2}{C}} - R^2 \qquad II,$$

worin $R^1$ und $R^2$ die vorgenannte Bedeutung besitzen, mit Oximethern der Formel

$$R^4 - \overset{NOR^3}{\overset{\|}{C}} - R^4 \qquad III,$$

worin die einzelnen Reste $R^4$ gleich oder verschieden sein können und jeweils einen aliphatischen Rest oder ein Wasserstoffatom bedeuten, in Gegenwart einer Säure als Katalysator umsetzt.

Die Umsetzung kann unter Verwendung von α,2,4-Trichloracetophenon und Acetonoxim-O-benzylether durch das folgende Formelschema wiedergegeben werden:

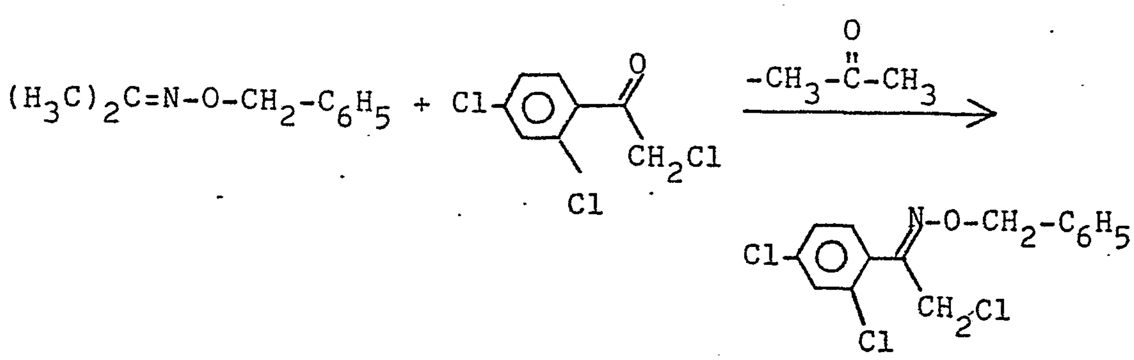

Im Hinblick auf den Stand der Technik liefert das Verfahren nach der Erfindung überraschend auf einfacherem und wirtschaftlicherem Wege eine große Zahl von α-Chlorketoximethern in guter Ausbeute, Raum-Zeit-Ausbeute und Reinheit, gerade auch im großtechnischen Maßstab und im kontinuierlichen Betrieb. Das erfindungsgemäße Verfahren weist eine Reihe von Vorteilen auf. So kann im allgemeinen der Oximether III als Lösungsmittel verwendet werden. Es sind keine Extraktionen zur Abtrennung wasserlöslicher Bestandteile notwendig, wodurch ein Trocknungsvorgang entfällt und Belastung des Abwassers vermieden wird. Hohe Ausbeuten von 80 % und mehr, verbunden mit der Einfachheit der Reaktionsführung, machen das erfindungsgemäße Verfahren wirtschaftlich. Es ist überraschend, daß bei den erfindungsgemäßen Verfahrensbedingungen die -Chlorketoximether I in hohen Ausbeuten gebildet werden, da man im Hinblick auf den Stand der Technik erwarten mußte, dieses nur bei Isolierung und Reinigung des O-substituierten Hydroxylamins erreichen zu können. Es konnte auch nicht erwartet werden, daß gerade Säure sich als vorzüglicher Katalysator für die Reaktion erweisen würde.

Der Ausgangsstoff II kann mit dem Ausgangsstoff III in stöchiometrischer Menge oder im Überschuß, vorzugsweise in einem Verhältnis von 1 bis 1.5, insbesondere von 1 bis 1.1 Mol Ausgangsstoff III je Mol Ausgangsstoff II, umgesetzt werden. Bevorzugte Ausgangsstoffe II und III und dementsprechend bevorzugte Endstoffe I sind solche in deren Formeln $R^1$, $R^2$ und $R^3$ gleich oder verschieden sein können und jeweils einen Alkylrest mit 1 bis 18, insbesondere 1 bis 7 Kohlenstoffatomen, einen Alkenyl- oder Alkinylrest mit 2 bis 18, insbesondere 2 bis 7 Kohlenstoffatomen, oder einen Cycloalkylrest mit 5 bis 8 Kohlenstoffatomen bedeuten, $R^1$ und $R^2$ auch jeweils einen, gegebenenfalls durch Bromatome oder Chloratome substituierten

Phenylrest bezeichnen, oder $R^1$ und ein Rest $R^2$ zusammen mit den beiden benachbarten Kohlenstoffatomen für Glieder eines alicyclischen, 5- oder 6-gliedrigen Ringes stehen können, $R^3$ auch einen Aralkylrest mit 7 bis 12 Kohlenstoffatomen bedeutet, $R^2$ auch für ein Wasserstoffatom steht, die einzelnen Reste $R^4$ gleich oder verschieden sein können und jeweils einen Alkylrest mit 1 bis 10 Kohlenstoffatomen, insbesondere 1 bis 4 Kohlenstoffatomen, oder ein Wasserstoffatom bedeuten. Die vorgenannten Reste können mit unter den Reaktionsbedingungen inerten Gruppen oder Atome, z.B. Chloratome, Bromatome, Alkylreste, Alkylgruppen mit 1 bis 4 Kohlenstoffatomen, substituiert sein. Die Reste $R^4$ sind zweckmäßig so zu wählen, daß die im Laufe der Umsetzung gebildete Carbonylverbindung rasch aus dem Reaktionsmedium entfernt werden kann. Dies kann z.B. durch eine Destillation erreicht werden. Die so gewonnene Carbonylverbindung wird dann durch Kondensation mit Hydroxylamin und anschließende Alkylierung wieder in den Oxim-O-ether der Formel III überführt. In einer bevorzugten Ausführungsform liegt der Siedepunkt der dem Oxim-O-ether der Formel III zugrundeliegenden Carbonylverbindung niedriger als der Siedepunkt des Ketons der Formel II.

So kommen als Ausgangsstoffe II in Betracht: α'-Chloraceton und α'-Methyl-, α-Ethyl-, α'-Propyl-, α'-Isopropyl-, α'-Butyl-, α'-Isobutyl-, α'-sek.-Butyl-, α'-tert.-Butyl-, α'-Cyclohexyl-, α'-Cyclopentyl-, α'-Phenyl-, α'-2-Chlorphenyl-, α'-3-Chlorphenyl-, α'-4-Chlorphenyl-, α'-2,4-Dichlorphenyl-, α'-2,6-Dichlorphenyl-, α'-3,5-Dichlorphenyl-α-chlormethylketon; entsprechende in α'-Stellung in vorgenannter Weise substituierte und in α-Stellung einfach oder gleich oder unterschiedlich zweifach durch die Methyl-, Ethyl-, Propyl-, Isopropyl-, Butyl-, Isobutyl-, sek.-Butyl-, tert.-Butyl-, Cyclohexyl-, Cyclopentyl-, Phenyl-,

2-Chlorphenyl-, 3-Chlorphenyl-, 4-Chlorphenyl-, 2,4-Dichlorphenyl-, 2,6-Dichlorphenyl-, 3,5-Dichlorphenyl-gruppen substituierte -Chlormethylketone; 1-Chlor-cyclohexanon, 1-Chlor-cyclopentanon, 1-Chlor-1-methylcyclohexanon.

Als Ausgangsstoffe III kommen in Betracht: Formaldoxim-, Acetaldoxim-, Acetonoxim-O-methylether; entsprechende Oxim-O-ether von Aldehyden und Ketonen, bei denen $R^4$ jeweils ein Wasserstoffatom und eine Ethyl-, Propyl-, Isopropyl-, Butyl-, Isobutyl-, sek.-Butyl-, tert.-Butyl-gruppe oder beide $R^4$ gleiche oder unterschiedliche vorgenannte Alkylgruppen tragen; die Ethyl-, Propyl-, Isopropyl-, Butyl-, Isobutyl-, sek.-Butyl-, tert.-Butyl-, Cyclohexyl-, Cyclopentyl-, Benzyl-, Phenylethyl-, Phenylpropyl-O-ether vorgenannter Oxime.

Die Umsetzung wird im allgemeinen bei einer Temperatur von 50 bis 200°C, vorzugsweise von 80 bis 150°C, insbesondere von 90 bis 130°C, mit Unterdruck, Überdruck oder drucklos, durchgeführt. Zweckmäßig ist die Reaktionstemperatur höher als der Siedepunkt der dem Oximether der Formel III zugrundeliegenden Carbonylverbindung. Bei Verwendung von Acetonoxim-O-ethern als Generator für O-substituierte Hydroxylamine ist der Temperaturbereich von 90 bis 150°C besonders bevorzugt.

Das erfindungsgemäße Verfahren wird im allgemeinen ohne Lösungsmittel durchgeführt. Sind jedoch beide Reaktionspartner fast oder nicht vollständig ineinander löslich oder erfordert der hohe Siedepunkt der im Laufe der Umsetzung gebildeten Carbonylverbindung eine Azeotropdestillation, so wird ein geeignetes, inertes Lösungsmittel, z.B. n-Butanol, n-Propanol, i-Propanol, Ethanol verwendet.

Die Umsetzung wird in Gegenwart von Säure als Katalysator, vorteilhaft mit einer Menge von 0.001 bis 0.25, insbesondere von 0.1 bis 0.2 Äquivalenten Säure, bezogen auf 1 Mol Ausgangsstoff II, durchgeführt. Es können anorganische oder organische Säuren verwendet werden. Anstelle einbasischer Säuren können auch äquivalente Mengen mehrbasischer Säuren zur Anwendung gelangen. Beispielsweise sind folgende Säuren geeignet: Chlorwasserstoff, Bromwasserstoff, Perchlorsäure, Schwefelsäure, Phosphorsäure, Salpetersäure, Sulfonsäuren wie Benzol- und p-Toluolsulfonsäure; Bor enthaltende Säuren wie Borsäure, Borfluorwasserstoffsäure; aliphatische Carbonsäuren wie Chloressigsäure, Dichloressigsäure, Trichloressigsäure, Oxalsäure, Ameisensäure, Cyanessigsäure, Essigsäure, Propionsäure, Buttersäure, Isobuttersäure, 3,5,5,-Trimethylhexansäure, 2-Ethylhexancarbonsäure, α-Ethylbuttersäure, 2-Methylbutansäure, Glykolsäure, Milchsäure, Monobromessigsäure, Trimethylessigsäure, α- bzw. ß-Chlorpropionsäure, Bernsteinsäure, Isovaleriansäure, Valeriansäure, Glutarsäure, Adipinsäure, Maleinsäure; cycloaliphatischen, araliphatischen, aromatischen Carbonsäuren wie Benzoesäure, 2,3-, 2,4-, 2,5-, 2,6-Dimethylbenzoesäure, o-, m- bzw. p-Aminobenzoesäure, o-, m- bzw. p-Oxybenzoesäure, Mellithsäure, Phenylpropionsäure, o-, m- bzw. p-Chlorbenzoesäure, Cyclohexancarbonsäure, Cyclopentancarbonsäure, Phenylessigsäure, α- bzw. ß-Naphthoesäure, 2,3-Oxynaphthoesäure, Zimtsäure, Naphthalin-1,8-dicarbonsäure, Phthalsäure, o-, m- bzw. p-Toluylsäure, o-, m- bzw. p-Nitrobenzoesäure, Isophthalsäure, Terephthalsäure; heterocyclische Carbonsäuren wie Imidazol-4-carbonsäure, Nicotinsäure, Pyrazincarbonsäure, Pyridin-o-carbonsäure, Chinolin-2-carbonsäure, Pyrrol-2-carbonsäure; Nitrilo-triessigsäure, Diglykolsäure, Thiodiglykolsäure, Sulfondiessigsäure; saure Ionenaustauscher; oder entsprechende Gemische. Die Säuren können in konzentrierter Form, im Gemisch miteinander und/oder mit einem Lösungsmittel, insbesondere Wasser, angewendet

werden. Bei verdünnten, wäßrigen Säuren sind 30 bis 96-gewichtsprozentige Säuren, z.B. 30 bis 35-gewichtsprozentige Salzsäure, oder 50 bis 96-gewichtsprozentige Schwefelsäure, vorteilhaft. Bevorzugt sind Salzsäure, Schwefelsäure, Phosphorsäure, p-Toluolsulfonsäure, Ameisensäure, Essigsäure. Der pH der Behandlung beträgt zwischen 1 und 7, vorzugsweise von 2 bis 5, insbesondere von 3 bis 4.5.

In einer bevorzugten Ausführungsform wird das erfindungsgemäße Verfahren in Gegenwart einer Lewis-Säure durchgeführt. Unter Lewis-Säuren werden hier elektrophile Stoffe mit unvollständiger Elektronenkonfiguration verstanden, die ein Elektronenpaar einer Base aufnehmen können. Bezüglich der Definition von Lewis-Säuren wird auf Houben-Weyl, Methoden der organischen Chemie, Band 4/2, Seite 6, und Rodd, Chemistry of Carbon Compounds, Band IA, Seite 103 (Elsevier Publ. Co. N.Y. 1951) verwiesen. Es kommen zweckmäßig als Lewis-Säuren Halogenide von Metallen der 2. bis 6. Gruppe des Periodischen Systems wie Zink-, Bor-, Aluminium-, Zinn-, Titan-, Antimon-, Wismut-, Molybdän-, Wolfram-chlorid, Aluminiumbromid und Bortrifluorid in Frage. Die Lewis-Säure kann man ebenfalls in Gestalt ihrer Komplexe, z.B. von Bortrifluorid-ätherat, -dihydrat; -äthylalkoholat und andere -alkoholaten; Fluorborsäure, Borfluorid-essigsäure, -diessigsäure, -phosphorsäure; Bortrichlorid-Komplexverbindungen mit Phosphortrichlorid und Phosphoroxychlorid verwenden.

Daneben kommen auch Lewis-Säuren und ihre Komplexe wie Zink- oder Zinnacetat und ihre Glycerinkomplexverbindungen, Eisen-II-sulfat, Zinnsulfat; Borsäureester, z.B. der Methylester, in Frage.

Die Reaktion kann wie folgt durchgeführt werden: Ein Gemisch von Ausgangsstoff II, Ausgangsstoff III, Katalysator,

gegebenenfalls zusammen mit Lösungsmittel, wird während 4 bis 8 Stunden bei der Reaktionstemperatur gehalten. Dann wird aus dem Reaktionsgemisch der Endstoff in üblicher Weise, z.B. durch Destillation, abgetrennt. Bei der Durchführung des erfindungsgemäßen Verfahrens setzt man vorzugsweise das α-Chlorketon II und den Oximether III ein, versetzt mit Katalysator und erhitzt. Die im Laufe der Reaktion gebildete Carbonylverbindung wird zweckmäßig kontinuierlich abdestilliert.

Die nach dem Verfahren der Erfindung herstellbaren α-Chlorketoximether I sind wertvolle Ausgangsstoffe für die Herstellung von Farbstoffen, Pharmazeutika und Schädlingsbekämpfungsmitteln. Die erfindungsgemäßen α-Chlorketoximether der Formel I sind wertvolle Zwischenprodukte für die Synthese von fungizid wirksamen -Azolylketoximethern der Formel IV

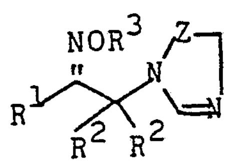

IV,

in der Z für CH oder N steht und $R^1$ und $R^2$ die in Formel I angegebenen Bedeutungen haben. Fungizide Wirkstoffe der Formel IV sind Gegenstand der DE-OS 27 23 942 und der DE-OS 26 57 578. Für ihre Herstellung wird die Umsetzung der entsprechenden Phenacylchloride oder -bromide (Halogenketone) mit den Azolen gemäß DE-OS 24 31 407 und die anschließende Oximierung und Verätherung empfohlen. Demgegenüber führt die Umsetzung der gut zugänglichen α-Chlorketoximether der Formel I mit dem Azolen zu befriedigenderen Ausbeuten, abgesehen davon, daß die α-Chlorketoximether für diesen Zweck nicht weiter gereinigt zu werden brauchen, das heißt, sie können ohne weitere Reinigung

mit 1,2,4-Triazol oder Imidazol zu den fungiziden Wirkstoffen der Formel IV umgesetzt werden.

Die Umsetzung wird bei einer Temperatur im Bereich zwischen 20 und 120°C in einem inerten organischen Lösungsmittel durchgeführt. Geeignet sind Alkohole, wie Methanol oder Ethanol, Nitrile, wie Acetonitril, Amide, wie Dimethylformamid, Diemethylacetamid. Auch Gemische dieser Lösungsmittel können verwendet werden.

Zur Bindung des bei der Umsetzung entstehenden Chlorwasserstoffs wird dem Reaktionsgemisch eine basische Substanz, beispielsweise ein Alkalimetallcarbonat, wie Natriumcarbonat oder Kaliumcarbonat, ein Alkoholat, wie Natriummethylat, Natriumethylat, Natriumhydrid, Butyllithium, ein Amin, wie Diisopropylethylamin, ein Lithiumamid, wie Lithiumdiisopropylamid, zugegeben. Der Chlorwasserstoff kann auch durch einen Überschuß an Imidazol oder 1,2,4-Triazol gebunden werden.

Die Reaktionspartner werden in stöchiometrischen Mengen eingesetzt. Vorzugsweise arbeitet man mit einem Überschuß an 1,2,4-Triazol bzw. Imidazol von 20 bis 200 Mol%.

Die in den folgenden Beispielen aufgeführten Teile bedeuten Gewichtsteile.

Beispiel 1

9,3 Teile Chloraceton, 24,5 Teile Acetonoxim-O-benzylether und 1 Teil konzentrierte Salzsäure werden 8 Stunden auf 150°C erhitzt. Es destillieren 5,2 Teile Aceton (90 % der Theorie) ab. Man erhält 18,2 Teile (92 % der Theorie) α-Chloracetonoxim-O-benzylether vom Kp. 128-130°C/0.01mbar.

Beispiel 2

53,5 Teile 3-Chlorbutanon-2, 122 Teile Acetonoximbenzylether und 5 Teile konzentrierte Salzsäure werden 8 Stunden auf 150°C erhitzt. Es destillieren 27,8 Teile Aceton (96 % der Theorie) ab. Der Rückstand wird im Vakuum von leichtflüchtigen Bestandteilen befreit. Man erhält 95,6 Teile (90 % der Theorie) 3-Chlorbutanon-2-oxim-O-benzylether vom Kp. 135--137°C/0.05mbar.

Beispiel 3

Man setzt analog Beispiel 1 die folgenden Ausgangsstoffe um: 13,3 Teile 2-Chlorcyclohexanon, 24,5 Teile Acetonoxim--O-benzylether, 1 Teil konzentrierte Salzsäure. Man erhält 15,2 Teile (64 % der Theorie) 2-Chlorcyclohexanonoxim-O--benzylether vom Kp. 152-153°C/0.02mbar.

Beispiel 4

Man setzt analog Beispiel 1 die folgenden Ausgangsstoffe um: 44,7 Teile α,2,4-Trichloracetophenon, 35,9 Teile Acetonoxim-O-benzylether, 0,4 Teile p-Toluolsulfonsäure. Man erhält 64,4 Teile (98 % der Theorie) α,2,4-Trichloracetophenonoxim-O-benzylether vom Kp. 145°C/5-$10^3$mbar.

Beispiel 5

Man setzt analog Beispiel 1 die folgenden Ausgangsstoffe um: 10,7 Teile 3-Chlorbutanon-2, 17 Teile Acetonoxim-O-alkylether, 2 Teile konzentrierte Salzsäure. Man erhält 10,7 Teile (66 % der Theorie) 3-Chlorbutanon-2-oxim-O-allylether vom Kp. 117119°C/0.1mbar.

## Patentanspruch

Verfahren zur Herstellung von α-Chlorketoximethern der Formel

$$R^1 - \overset{\overset{\displaystyle NOR^3}{\|}}{C} - \underset{\underset{\displaystyle Cl}{|}}{\overset{\overset{\displaystyle R^2}{|}}{C}} - R^2 \qquad I,$$

worin $R^1$, $R^2$ und $R^3$ gleich oder verschieden sein können und jeweils einen aliphatischen oder cycloaliphatischen Rest bedeuten, $R^1$ und $R^2$ auch jeweils einen gegebenenfalls durch Halogenatome substituierten Arylrest bezeichnen oder $R^1$ und ein Rest $R^2$ zusammen mit den beiden benachbarten Kohlenstoffatomen für Glieder eines alicyclischen Ringes stehen können, $R^3$ auch einen araliphatischen Rest bedeutet, $R^2$ auch für ein Wasserstoffatom steht, <u>dadurch gekennzeichnet</u>, daß man α-Chlorketone der Formel

$$R^1 - \overset{\overset{\displaystyle O}{\|}}{C} - \underset{\underset{\displaystyle Cl}{|}}{\overset{\overset{\displaystyle R^2}{|}}{C}} - R^2 \qquad II,$$

worin $R^1$ und $R^2$ die vorgenannte Bedeutung besitzen, mit Oximethern der Formel

$$R^4 - \overset{\overset{\displaystyle NOR^3}{\|}}{C} - R^4 \qquad III,$$

worin die einzelnen Reste $R^4$ gleich oder verschieden sein können und jeweils einen aliphatischen Rest oder ein Wasserstoffatom bedeuten, in Gegenwart einer Säure als Katalysator umsetzt.

378/80 WB/Ws 25.11.1980

0052742

Europäisches Patentamt

**EUROPÄISCHER RECHERCHENBERICHT**

Nummer der Anmeldung

EP 81 10 8209

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| X | DE - B - 1 155 440 (COMMERCIAL SOLVENTS CORPORATION)<br>* Ansprüche und Beschreibung *<br>-- | einzige | C 07 C 131/00 |
| X | JOURNAL OF THE AMERICAN CHEMICAL SOCIETY, Band 78, Nr. 11, June 8, 1956,<br>Washington D.C. (US)<br>M.S. NEWMAN et al.: "α-(2,4,5,7-Tetranitro-9-fluorenylideneamino-öxy)-propionic acid, a new reagent for resolution by complex formation[1]"<br>Seiten 2469-2473<br>* Seite 2470, Spalte 1 *<br>---- | einzige | RECHERCHIERTE SACHGEBIETE (Int. Cl.³)<br>C 07 C 131/00 |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 05-03-1982 | GAUTIER |